# EUROPÄISCHE PATENTSCHRIFT

(11) **EP 1 879 560 B1**
(45) Veröffentlichungstag und Bekanntmachung des Hinweises auf die Patenterteilung: **27.08.2014**
(21) Anmeldenummer: 06753441.2
(22) Anmeldetag: 28.04.2006
(51) Int. Cl.: A61K 9/70, A61K 31/57

(54) **FESTES TRANSDERMALES THERAPEUTISCHES SYSTEM MIT UV-ABSORBER**
SOLID TRANSDERMAL THERAPEUTIC SYSTEM COMPRISING UV ABSORBER
SYSTEME THERAPEUTIQUE TRANSDERMIQUE SOLIDE EQUIPE D'ABSORBEURS D'U.V.

(30) Priorität: 02.05.2005 EP 05009579
(43) Veröffentlichungstag der Anmeldung: 23.01.2008
(73) Patentinhaber: Bayer Intellectual Property GmbH, 40789 Monheim (DE)
(72) Erfinder: LANGGUTH, Thomas, 07751 Jena (DE); BRACHT, Stefan, 16548 Glienicke-Nordbahn (DE)
(74) Vertreter: BIP Patents
(86) Internationale Anmeldenummer: PCT/EP2006/003959
(87) Internationale Veröffentlichungsnummer: WO 2006/117139

(56) Entgegenhaltungen:
- EP-A- 1 269 999
- EP-A- 1 452 173
- WO-A1-2005/002482
- CHATELAIN E ET AL: "PHOTOSTABILIZATION OF BUTYL METHOXYDIBENZOYLMETHANE (AVOBENZONE) AND ETHYLHEXYL METHOXYCINNAMATE BY BIS-ETHYLHEXYLOXYPHENOL METHOXYPHENYL TRIAZINE (TINOSORB S), A NEW UV BROADBAND FILTER" PHOTOCHEMISTRY AND PHOTOBIOLOGY, OXFORD, GB, Bd. 74, Nr. 3, September 2001 (2001-09), Seiten 401-406, XP001057313 ISSN: 0031-8655

## Beschreibung

### Technisches Gebiet

Die Erfindung betrifft ein festes transdermales therapeutisches System mit UV-Absorber. Das UV stabile transdermales therapeutisches System (TTS) ist besonders ausgelegt für lichtempfindliche pharmazeutische Wirkstoffe.

Das erfindungsgemäße transdermale therapeutische System, gegebenenfalls ein Gestagen und/oder ein Estrogen enthaltend, eignet sich auch zur Fertilitätskontrolle.

### Stand der Technik

Es sind Versuche bekannt, lichtempfindliche Wirkstoffe, welche UV-A- und UV-B-Strahlen absorbieren, üblicherweise in Sonnencremes einzusetzen, wie von Briscart & Plaizier-Vercammen (Proc. 2nd World Meeting on Pharmaceutics, Biopharmaceutics and Pharmaceutical Technology, APGI/ APV, 1998, 1231-1232) beschrieben.

Ferner ist aus der Patentliteratur bekannt, mit lichtempfindlichen Wirkstoffen versehene transdermale therapeutische Systeme (TTS) mittels optisch auffallenden aluminisierten oder lackierten Abdeckfolien als Rückschicht des TTS zu schützen.

Die WO-A1-00/56289 beschreibt ein Verfahren zum Schutz therapeutischer Zubereitungen, Systeme oder deren Bestandteile, wobei ein jeweils spezifischer Schutz gegen Abbau durch schädliche Faktoren, wie Luftsauerstoff, Wasser und/oder Licht realisiert werden soll. Es werden elektromagnetische Wellen absorbierende bzw. reflektierende Lichtschutzsubstanzen verwendet, wobei Absorptions- bzw. Reflexionsmittel eingesetzt werden, deren Absorptions- bzw. Reflexionsspektrum denjenigen Wellenlängenbereich umfasst, der für die Instabitität des lichtempfindlichen Stoffes bzw. seiner Bestandteile verantwortlich ist. Als Abdeckfolie werden hier u.a. eingefärbte Kunststofffolien verwendet, am Beispiel vom 1,4-Dihydopyridinderivat Lacidipin aufgezeigt.

Die Einfärbung von hochflexiblen Kunststofffolien erweist sich als schwierig und bietet durch häufig auftretende Risse in der Farbschicht der Kunststofffolie keinen zuverlässigen Lichtschutz.

Ferner sind aus WO-A2-02/34200 transdermale therapeutische Systeme (TTS) bekannt, die aus einer wirkstoffhaltigen Polymermatrix und einer Rückschicht bestehen, wobei Polymermatrix und Rückschicht fest verbunden sind bzw. ein Laminat bilden und sowohl die Polymermatrix als auch die Rückschicht eine im UV-Bereich absorbierende, farblose Substanz enthalten, die keine eigene pharmakologische Wirkung aufweist.

In der EP-A1-1452173 sind transdermale therapeutische Systeme aufgezeigt, welche aus einer Rückschicht, mindestens einer wirkstoffhaltigen Matrix und wahlweise einer Abziehfolie besteht sowie einen UV-Absorber enthält, wobei zwischen der Rückschicht und der der Hautoberfläche am weitesten abgewendeten wirkstoffhaltigen Matrix mindestens eine UV-Absorber enthaltende Klebschicht vorgesehen ist und zwischen der den UV-Absorber enthaltenden Klebschicht und der der Hautoberfläche am weitesten abgewendeten wirkstoffhaltigen Matrix mindestens eine Trennschicht enthalten ist, die wirkstoffundurchlässig und für den UV-Absorber undurchlässig ist. Als UV-Absorber werden hier die UV-Absorber aus der Gruppe p-Aminobenzoesäure, Aminobenzoesäurederivat, vorzugsweise 4-Dimethylaminobenzoesäure-2-ethyl-hexylester und/oder 4-Bis-(polyethoxyl)aminobenzoesäure-polyethoxyethylester, Zimtsäure,Zimtsäure-Derivate, vorzugsweise 4-Methoxylzimt-säureisoamylester und/oder 4-Methoxyzimtsäure-2-ethylhexyl-ester, 3-Benzylidenbornan-2-on, Benzylidenbornan-2-on-Derivate, vorzugsweise 3-(4')-Methylbenzylinden-bornan-2-on, 3-(4-Sulfon)benzylidenbornan-2-on und/oder 3-(4'-Trimethyl-ammonium)benzylidenbornan-2-on-methylsulfat, Salicylsäurederivat, vorzugsweise 4-Isopropylbenzylsalicylat, Salicysäure-2-ethylhexylester, und/oder 3, 3, 5-Trimethyl-cyclohexylsalicylat, Benzotriazole, vorzugsweise 2-(5-chloro-2H-benzotriazole-2-yl)-6-(1,1-dimethylethyl)-4-methyl-phenol, 2, 4 ,6'-Trianilin-p-(carbo-2'-ethylhexyl-1'-oxy)-1, 3, 5-triazin, 3-Imidazol-4-yl-acrylsäure, 3-Imidazol-4-yl-.3-Imidazol-4-yl-acrylsäure-Ester, 2-Phenylenbenzimidazol-5-sulfonsäure und/oder ihre K-, Na- und Triethanolamin (=TEA)-Salze, 2-Cyan-3,3-diphenylacrylsäure, Terephthaloyliden-dicampher-sulfonsäure, Butylmethoxy-dibenzoyl-methan, Benzophenon und/oder Benzophenon-Derivate, vorzugsweise Benzophenon-3 und/oder Benzophenon-4 ausgewählt sein kann/können.

Nachteilig an den bekannten Lösungen ist,
- dass die durch den zugesetzten UV-Absorber erzeugte Schutzwirkung für den Wirkstoff unvollständig ist,
- dass durch die unvollständige Schutzwirkung teilweise höhere Konzentrationen der UV-Absorber eingesetzt werden müssen, die sich negativ auf die Hautverträglichkeit der TTS auswirken können.

### Darstellung der Erfindung

Aufgabe der Erfindung ist deshalb, eine mit einem lichtempfindlichen Wirkstoff versehene transdermal zu applizierende Arzneistoffzubereitung bereitzustellen, welche eine erhöhte Schutzwirkung für den Wirkstoff unter Verwendung einer möglichst geringen Konzentration des UV-Absorbers gewährleistet, die die vorher genannten Nachteile vermeidet.

Erfindungsgemäß wird die Aufgabe durch ein festes transdermales therapeutisches System mit UV-Absorber gelöst. Das UV stabile TTS besteht dabei in seiner Schichtfolge aus einer Rückschicht 1, mindestens einer wirkstoffhaltigen Matrix 2 und einer abziehbaren Schutzfolie 3 , wobei wahlweise zwischen der Rückschicht 1 und der wirkstoffhaltigen Matrix 2 eine Klebeschicht 4 und eine Trennschicht 5 eingebracht sein, und wobei die wirkstoffhaltige Matrix ein Gestagen enthält. In der Rückschicht 1 oder in die wirkstoffhaltige Matrix 2 oder in die Klebeschicht 4 sind UV-Absorber aus der Gruppe der Hydroxyphenyltriazine eingebettet.

Erfindungsgemäß kann der UV-Absorber 2,4-bis-i[4-(2-ethyl-hexyloxy)-2-hydrody]-phenylý-6-(4-methoxyphenyl)-(1,3,5)-triazin sein.

Ferner kann erfindungsgemäß im festen transdermalen therapeutischen System das Flächengewicht der Matrix 2, 30 bis 150 g/m² betragen. Bevorzugt ist dabei ein Flächengewicht von 50 bis 120 g/m² und besonders bevorzugt von 100g/m².

Auch kann beim erfindungsgemäßen festen transdermalen therapeutischen System das Flächengewicht der Klebschicht 4, 5 bis 50 g/m². betragen. Bevorzugt ist dabei ein Flächengewicht von 20 bis 30 g/m².

Erfindungsgemäß kann der UV-Absorber in der Klebschicht 4 in der Konzentration von 0,5 bis 5 % (m/m) in gelöster form vorliegen. Bevorzugt ist dabei eine Konzentration von 1,0 bis 4,0 % und besonders bevorzugt von 1,5 bis 3,0 %.

Ferner kann erfindungsgemäß im festen transdermalen therapeutischen System die Matrix 2 und/ oder die Klebschicht 4 selbstklebend ausgeführt sein und im wesentlichen aus Polymeren bestehen, die aus den Gruppen Polyisobutylen, Polybuten,Polyacrylat, Polydimethylsiloxan, Styrol-Isopren-Blockpolymerisat oder Polyisopren ausgewählt sind.

Auch kann im erfindungsgemäßen festen transdermalen therapeutischen System die Trennschicht 5 eine Schichtdicke von 4 bis 23 µm aufweisen. Bevorzugt dabei ist die Schichtdicke von 4 bis 10 µm.

Erfindungsgemäß kann im festen transdermalen therapeutischen System Trennschicht 5 wirkstoffundurchlässig und für den UV-Absorber undurchlässig sein.

Ferner kann erfindungsgemäß im festen transdermalen therapeutischen System die Trennschicht 5 aus einem Barrierepolymer bestehen. Bevorzugt ist dabei Polyethylenterephthalat oder Polyacrylnitril oder Polyvinylchlorid oder Polyvinylidenchlorid oder dessen Copolymeren oder Kolaminaten.

Auch kann im erfindungsgemäßen festen transdermalen therapeutischen System die Rückschicht 1 wirkstoffdurchlässig sein und aus Polypropylen, Polyethylen, Polyurethan, Ethylen-Vinylacetat-Copolymer oder einem Mehrschichtverbund aus diesen Materialien untereinander oder mit anderen Materialien bestehen.

Erfindungsgemäß kann/können im festen transdermalen therapeutischen System der/die UV-Absorber farblos oder gelblich sein.

Ferner kann das erfindungsgemäße feste transdermale therapeutisches System transparent oder schwach opak ist.

Der pharmazeutische Wirkstoffe ist ein Gestagen. Bevorzugt ist dabei Gestoden oder Levonorgestrel.

Ferner kann im erfindungsgemäßen festen transdermalen therapeutischen System zum Gestagen ein Estrogen zugefügt sein. Bevorzugt ist dabei Ethinylestradiol.

Das feste transdermale therapeutische System kann in der Fertilitätskontrolle eingesetzt werden.

Auch kann erfindungsgemäß das feste transdermale therapeutische System ohne eine die Wirkstofffreisetzung steuernde Membran ausgerüstet sein.

Das erfindungsgemäße transdermale therapeutische System besitzt gegegenüber herkömmlichen Systemen mit lichtempfindlichen Wirkstoffgehalt folgende Vorteile:
- Die durch den UV-Absorber aus der Gruppe der Hydroxyphenyltriazine bereitgestellte Schutzwirkung ist verstärkt ist und
- die für die Erreichung einer Schutzwirkung notwendige Konzentration des UV-Absorbers aus der Gruppe der Hydroxyphenyltriazine ist reduziert.
- Damit kann insbesondere das Risiko für eine mögliche Hautirritation vermieden bzw. reduziert werden.

### Ausführungsbeispiele

Die Erfindung wird durch nachfolgende Beispiele näher erläutert.

### Beispiel 1

Es wurden zwei Formulierungen eines lichtempfindlichen Wirkstoffes aus der Gruppe der Gestagene hergestellt. Formulierung 2 enthält eine Klebeschicht und eine Trennschicht, wobei die Klebeschicht 2,5 Gew.% einer UVabsorbierende Substanz aus der Klasse der Hydroxyphenyltriazine enthält. Formulierung 1 enthält keine Klebeschicht und Trennschicht und dient als Vergleichsformulierung. Beide Formulierungen enthalten eine wirkstoffhaltige Matrix mit einem lichtempfindlichem Gestagen und wurden mit einer Rückschicht aus Polyethylen ausgestattet, wodurch jeweils ein TTS erhalten wird. Die Formulierung 2 hat folgende Zusammensetzung:
1. wirkstoffhaltige Matrix:
   - 1,9 % Gestagen
   - 98,1 % Polyisobutylen basierendes Klebemittel
2. Klebeschicht:
   - 2,5 % Tinosorb®S
   - 97,2 % Polyisobutylen basierendes Klebemittel
      Tinosorb®S (Fa. CIBA, Lampertheim) ist ein UV-Absorber der Hydroxyphenyltriazine -Klasse.

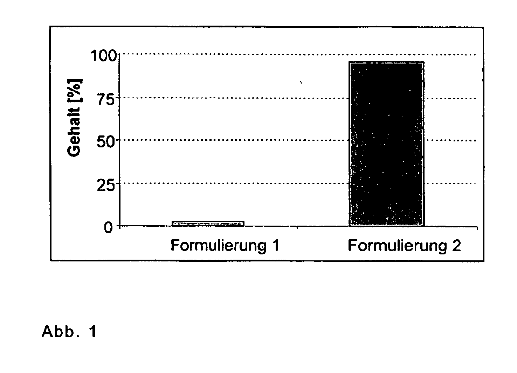

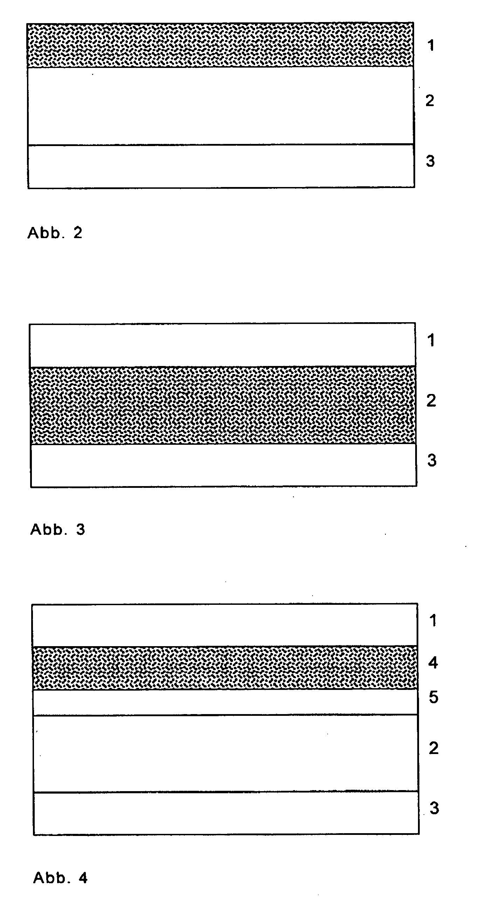

Zur Untersuchung der Lichtschutzwirkung wurden beide Formulierungen mit Licht mit einem UV-Spektrum von 300 - 800 nm über eine Dauer von bis zu 34h bestrahlt. Als Strahlungsquelle wurde eine Xenon-Lampe verwendet. Zwischen der Strahlungsquelle und den zu bestrahlenden Proben wurde eine Filtersystem (Typ: Suprax®-Filter) gesetzt, um die Bestrahlung unter realistischen Anwendungsbedingungen der TTS nachzuahmen. Anschließend wurde der Wirkstoffgehalt in den TTS bestimmt. Es zeigte sich, dass die TTS der Formulierung 2, die eine Klebeschicht mit UV-absorbierender Substanz und eine Trennschicht enthielt, nach Bestrahlung über 34 h noch mehr als 95% der ursprünglich eingesetzten Menge des lichtempfindlichen Wirkstoffes enthielten, wogegen die TTS der Formulierung 1 nach der Bestrahlung nur noch ca. 3 % der ursprünglich eingesetzten Menge des lichtempfindlichen Wirkstoffs enthielten (Abb.1). Dies zeigt, dass das erfindungsgemäße System unter realistischen Anwendungsbedingungen einen verbesserten Sonnenschutz aufweist, da die UV-Schutzwirkung des erfindungsgemäßen Systems (Formulierung 2) wesentlich höher als die des Vergleichssystems (Formulierung 1) war.

### Beispiel 2

Formulierung mit einem lichtempfindlichen Wirkstoffes aus der Gruppe der Gestagene mit jeweils einer Klebschicht und Trennschicht, bei dem die Trennschichte aus Polyethylenterephthalat (Hostaphan®, Fa. Mitsubishi Polyester, Wiesbaden) besteht.

Die Formulierung hat folgende Zusammensetzung:
1. wirkstoffhaltige Matrix:
   - 1,9 % Gestagen
   - 98,1 % Polyisobutylen basierendes Klebemittel
2. Klebeschicht 1 und 2:
   - 2,5 % Tinosorb®S
   - 97,5 % Polyacrylat basierendes Klebemittel

### Beispiel 3

Formulierung mit einem lichtempfindlichen Wirkstoffes aus der Gruppe der Gestagene mit jeweils zwei Klebschichten und Trennschichten, bei dem die Trennschichten aus Polyethylenterephthalat (Hostaphan®, Fa. Mitsubishi Polyester, Wiesbaden) bestehen.

Die Formulierung I hat folgende Zusammensetzung:
1. wirkstoffhaltige Matrix:
   - 1,9 % Gestagen
   - 98,1 % Polyisobutylen basierendes Klebemittel
2. Klebeschicht 1 und 2:
   - 3 % Tinuvin®400
   - 97 % Polyacrylat basierendes Klebemittel
      Tinuvin®400 (Fa. CIBA, Lampertheim) ist ein UV-Absorber der Hydroxyphenyltriazine -Klasse.

### Beispiel 4 bis 12

Formulierung mit einem lichtempfindlichen Wirkstoffe aus der Gruppe der Gestagene mit jeweils mindestens einer Klebschicht und Trennschicht, bei denen die wirkstoffhaltige Matrix analog zu den Beispielen 1 bis 3 ausgeführt ist und die Klebschicht ein Polyisobutylen basierendes Klebemittel enthält und die nachfolgend genannten Zusammensetzungen besitzt.

| Zusammensetzung der Klebschicht | Beispiel | | | | | | | | |
|---|---|---|---|---|---|---|---|---|---|
| | 4 | 5 | 6 | 7 | 8 | 9 | 10 | 11 | 12 |
| Tinosorb^{®}S [%] | 2 | 2 | 2 | 3 | 3 | 3 | 4 | 4 | 4 |
| Polyisobutylen basierendes Klebemittel [%] | 98 | 98 | 98 | 97 | 97 | 97 | 96 | 96 | 96 |
| Flächengewicht [g/m²] | 20 | 30 | 50 | 20 | 30 | 50 | 20 | 30 | 50 |

### Beispiel 13 bis 21

Formulierung mit einem lichtempfindlichen Wirkstoffes aus der Gruppe der Gestagene mit jeweils mindestens einer Klebschicht und Trennschicht, bei denen die wirkstoffhaltige Matrix analog zu den Beispielen 1 bis 3 ausgeführt ist und die Klebschicht eine Polyacrylat basierendes Klebemittel enthält und die nachfolgend genannten Zusammensetzungen besitzt.

| Zusammensetzung der Klebschicht | Beispiel | | | | | | | | |
|---|---|---|---|---|---|---|---|---|---|
| | 13 | 14 | 15 | 16 | 17 | 18 | 19 | 20 | 21 |
| Tinosorb^{®}S [%] | 2 | 2 | 2 | 3 | 3 | 3 | 4 | 4 | 4 |
| Polyacrylat basierendes Klebemittel [%] | 98 | 98 | 98 | 97 | 97 | 97 | 96 | 96 | 96 |
| Flächengewicht [g/m²] | 20 | 30 | 50 | 20 | 30 | 50 | 20 | 30 | 50 |

## Patentansprüche

1. Festes transdermales therapeutisches System mit UV-Absorber, dessen Schichtenfolge aus mindestens drei Schichten besteht, wobei diese Schichtenfolge am weitesten von der Haut entfernt beginnt mit einer Rückschicht (1),
einer mindestens einschichtigen wirkstoffhaltigen Matrix (2),
einer abziehbaren Schutzfolie (3) und
wahlweise einer zwischen Rückschicht (1) und wirkstoffhattiger Matrix (2) eingebrachten Klebeschicht (4) und
einer zur wirkstoffhaltigen Matrix (2) hin folgenden Trennschicht (5), wobei der oder die UV-Absorber aus der Gruppe der Hydroxyphenyltriazine eingebettet ist bzw. sind in die Rückschicht (1) oder in die wirkstoffhaltige Matrix (2) oder in die Klebeschicht (4) und wobei die wirkstoffhaltige Matrix ein Gestagen enthält.

2. Festes transdermales therapeutisches System nach Anspruch 1, **dadurch gekennzeichnet, dass** der UV-Absorber 2,4-Bis-[4-(2-ethylhexyloxy)-2-hydroxy]-phenyl-6-(4-methoxyphenyl)-(1,3,5)-triazin ist.

3. Festes transdermales therapeutisches System nach mindestens einem der vorhergehenden Ansprüche, **dadurch gekennzeichnet, dass** das Gestagen vorzugsweise Gestoden oder Levonorgestrel ist.

4. Festes transdermales therapeutisches System nach den Ansprüchen 3,
**dadurch gekennzeichnet, dass** ein Estrogen, vorzugsweise Ethinylestradiol enthalten ist.

5. Festes transdermales therapeutisches System nach mindestens einem der vorhergehenden Ansprüche 3 und 4, **dadurch gekennzeichnet, dass** das Flächengewicht der Matrix (2) 30 bis 150 g/m², vorzugsweise 50 bis 120 g/m² beträgt.

6. Festes transdermales therapeutisches System nach mindestens einem der vorhergehenden Ansprüche 4 bis 5, **dadurch gekennzeichnet, dass** das Flächengewicht der Klebschicht (4) 5 bis 50 g/m², vorzugsweise 20 bis 30 g/m² beträgt.

7. Festes transdermales therapeutisches System nach Anspruch 6,
**dadurch gekennzeichnet, dass** in der Klebschicht (4) mindestens ein UV-Absorber in einer Konzentration von 0,5 bis 5 % (m/ m), vorzugsweise 1,0 bis 4,0 % (m/ m) in gelöster Form vorliegt.

8. Festes transdermales therapeutisches System nach mindestens einem der vorhergehenden Ansprüche 4 bis 7 , **dadurch gekennzeichnet, dass** die Matrix (2) und/ oder die Klebschicht (4) selbstklebend ausgeführt ist/ sind und im wesentlichen aus Polymeren besteht, die aus den Gruppen Polyisobutylen, Polybuten, Polyacrylat, Polydimethylsiloxan, Styrol-Isopren-Blockpolymerisat oder Polyisopren ausgewählt sind.

9. Festes transdermales therapeutisches System nach mindestens einem der vorhergehenden Ansprüche 4 bis 8, **dadurch gekennzeichnet, dass** die Trennschicht (5) eine Schichtdicke von 4 bis 23 µm, vorzugsweise von 4 bis 10 µm, aufweist.

10. Festes transdermales therapeutisches System nach Anspruch 9, **dadurch gekennzeichnet, dass** die Trennschicht (5) wirkstoff-undurchlässig und für den UV-Absorber undurchlässig ist.

11. Festes transdermales therapeutisches System nach mindestens einem der vorhergehenden Ansprüche 4 bis 10, **dadurch gekennzeichnet, dass** die Trennschicht (5) aus einem Barrierepolymer besteht, vorzugsweise aus Polyethylenterephthalat, Polyacrylnitril, Polyvinylchlorid, Polyvinylidenchlorid oder dessen Copolymeren oder Kolaminaten.

12. Festes transdermales therapeutisches System nach mindestens einem der vorhergehenden Ansprüche 4 bis 11, **dadurch gekennzeichnet, dass** die Rückschicht (1) wirkstoffdurchlässig ist und vorzugsweise aus Polypropylen, Polyethylen, Polyurethan, Ethylen-Vinylacetat-Copolymer oder einem Mehrschichtverbund aus diesen Materialien untereinander oder mit anderen Materialien besteht.

13. Festes transdermales therapeutisches System nach mindestens einem der vorhergehenden Ansprüche 4 bis 12, **dadurch gekennzeichnet, dass** der UV-Absorber farblos oder gelblich ist.

14. Festes transdermales therapeutisches System nach mindestens einem der vorhergehenden Ansprüche 4 bis 13, **dadurch gekennzeichnet, dass** das transdermale therapeutische System transparent oder schwach opak ist.

## Claims

1. Solid transdermal therapeutic system with UV absorber, whose sequence of layers consists of at least three layers, this sequence of layers starting furthest away from the skin with a backing layer (1),
an at least monolayer active ingredient-containing matrix (2),
a detachable protective film (3) and
optionally, introduced between backing layer (1) and active ingredient-containing matrix (2), an adhesive layer (4) and
a separating layer (5) following the active ingredient-containing matrix (2), the UV absorber(s) from the group of hydroxyphenyltriazines being embedded in the backing layer (1) or in the active ingredient-containing matrix (2) or in the adhesive layer (4) and the active ingredient-containing matrix containing a progestogen.

2. Solid transdermal therapeutic system according to Claim 1, **characterized in that** the UV absorber is 2,4-bis-[4-(2-ethylhexyloxy)-2-hydroxy]phenyl-6-(4-methoxyphenyl)-(1,3,5)-triazine.

3. Solid transdermal therapeutic system according to at least one of the preceding claims, **characterized in that** the progestogen is preferably gestodene or levonorgestrel.

4. Solid transdermal therapeutic system according to Claim 3, **characterized in that** an estrogen, preferably ethynylestradiol, is present.

5. Solid transdermal therapeutic system according to at least one of the preceding Claims 3 and 4, **characterized in that** the weight per unit area of the matrix (2) is from 30 to 150 g/m², preferably 50 to 120 g/m².

6. Solid transdermal therapeutic system according to at least one of the preceding Claims 4 and 5, **characterized in that** the weight per unit area of the adhesive layer (4) is from 5 to 50 g/m², preferably 20 to 30 g/m².

7. Solid transdermal therapeutic system according to Claim 6, **characterized in that** at least one UV absorber is present in a concentration of from 0.5 to 5% (m/m), preferably 1.0 to 4.0% (m/m), in dissolved form in the adhesive layer (4).

8. Solid transdermal therapeutic system according to at least one of the preceding Claims 4 to 7, **characterized in that** the matrix (2) and/or the adhesive layer (4) is/are designed to be self-adhesive and substantially consists of polymers which are selected from the groups of polyisobutylene, polybutene, polyacrylate, polydimethylsiloxane, styrene-isoprene block polymer or polyisoprene.

9. Solid transdermal therapeutic system according to at least one of the preceding Claims 4 to 8, **characterized in that** the separating layer (5) has a layer thickness of from 4 to 23 µm, preferably from 4 to 10 µm.

10. Solid transdermal therapeutic system according to Claim 9, **characterized in that** the separating layer (5) is impermeable to active ingredient and impermeable to the UV absorber.

11. Solid transdermal therapeutic system according to at least one of the preceding Claims 4 to 10, **characterized in that** the separating layer (5) consists of a barrier polymer, preferably of polyethylene terephthalate, polyacrylonitrile, polyvinyl chloride, polyvinylidene chloride or its copolymers or colaminates.

12. Solid transdermal therapeutic system according to at least one of the preceding Claims 4 to 11, **characterized in that** the backing layer (1) is permeable to active ingredient and preferably consists of polypropylene, polyethylene, polyurethane, ethylene-vinyl acetate copolymer or a multilayer composite of these materials with one another or with other materials.

13. Solid transdermal therapeutic system according to at least one of the preceding Claims 4 to 12, **characterized in that** the UV absorber is colourless or yellowish.

14. Solid transdermal therapeutic system according to at least one of the preceding Claims 4 to 13, **characterized in that** the transdermal therapeutic system is transparent or slightly opaque.

## Revendications

1. Système thérapeutique transdermique solide présentant un/des absorbant(s) des UV, dont la succession de couches est constituée par au moins trois couches, cette succession de couches commençant, à la plus grande distance de la peau, par une couche arrière (1),
une matrice au moins monocouche, contenant une/des substance(s) active(s) (2),
une feuille de protection pouvant être retirée (3) et au choix une couche adhésive (4) introduite entre la couche arrière (1) et la matrice contenant une/des substance(s) active(s) (2) et
une couche de séparation (5) consécutive vers la matrice contenant une/des substance(s) active(s) (2), le ou les absorbants des UV du groupe des hydroxyphényltriazines étant enrobé(s) dans la couche arrière (1) ou dans la matrice contenant une/des substance (s) active(s) (2) ou dans la couche adhésive (4) et la matrice contenant une/des substance(s) active(s) contenant un gestagène.

2. Système thérapeutique transdermique solide selon la revendication 1, **caractérisé en ce que** l'absorbant des UV est la 2,4-bis-[4-(2-éthylhexyloxy)-2-hydroxy]-phényl-6-(4-méthoxyphényl)-(1,3,5)-triazine.

3. Système thérapeutique transdermique solide selon au moins l'une quelconque des revendications précédentes, **caractérisé en ce que** le gestagène est de préférence le gestodène ou le lévonorgestrel.

4. Système thérapeutique transdermique solide selon la revendication 3, **caractérisé en ce qu'**un estrogène, de préférence de l'éthinylestradiol, est contenu.

5. Système thérapeutique transdermique solide selon au moins l'une quelconque des revendications précédentes 3 et 4, **caractérisé en ce que** le poids surfacique de la matrice (2) vaut 30 à 150 g/m², de préférence 50 à 120 g/m².

6. Système thérapeutique transdermique solide selon au moins l'une quelconque des revendications précédentes 4 à 5, **caractérisé en ce que** le poids surfacique de la couche adhésive (4) vaut 5 à 50 g/m², de préférence 20 à 30 g/m².

7. Système thérapeutique transdermique solide selon la revendication 6, **caractérisé en ce qu'**au moins un absorbant des UV se trouve dans la couche adhésive (4) en une concentration de 0,5 à 5% (M/M), de préférence de 1,0 à 4,0% (M/M) sous forme dissoute.

8. Système thérapeutique transdermique solide selon au moins l'une quelconque des revendications précédentes 4 à 7, **caractérisé en ce que** la matrice (2) et/ou la couche adhésive (4) est/sont apprêtée(s) de manière autoadhésive et essentiellement constituée(s) par des polymères qui sont choisis dans le groupe formé par le polyisobutylène, le polybutène, le polyacrylate, le polydiméthylsiloxane, les copolymères à blocs de styrène-isoprène ou le polyisoprène.

9. Système thérapeutique transdermique solide selon au moins l'une quelconque des revendications précédentes 4 à 8, **caractérisé en ce que** la couche de séparation (5) présente une épaisseur de couche de 4 à 23 µm, de préférence de 4 à 10 µm.

10. Système thérapeutique transdermique solide selon la revendication 9, **caractérisé en ce que** la couche de séparation (5) est imperméable à la/aux substance(s) active(s) et imperméable aux absorbants des UV.

11. Système thérapeutique transdermique solide selon au moins l'une quelconque des revendications précédentes 4 à 10, **caractérisé en ce que** la couche de séparation (5) est constituée par un polymère formant une barrière, de préférence de poly(téréphtalate d'éthylène), de polyacrylonitrile, de poly(chlorure de vinyle), de poly(chlorure de vinylidène) ou leurs copolymères ou costratifiés.

12. Système thérapeutique transdermique solide selon au moins l'une quelconque des revendications précédentes 4 à 11, **caractérisé en ce que** la couche arrière (1) est perméable à la/aux substance (s) active(s)et est de préférence constituée de polypropylène, de polyéthylène, de polyuréthane, de copolymère d'éthylène-acétate de vinyle ou d'un composite multicouche de ces matériaux les uns avec les autres ou avec d'autres matériaux.

13. Système thérapeutique transdermique solide selon au moins l'une quelconque des revendications précédentes 4 à 12, **caractérisé en ce que** l'absorbant des UV est incolore ou jaunâtre.

14. Système thérapeutique transdermique solide selon au moins l'une quelconque des revendications précédentes 4 à 13, **caractérisé en ce que** le système thérapeutique transdermique est transparent ou faiblement opaque.
